# EUROPÄISCHE PATENTSCHRIFT

(11) **EP 1 857 430 B1**
(45) Veröffentlichungstag und Bekanntmachung des Hinweises auf die Patenterteilung: **21.12.2016**
(21) Anmeldenummer: 06010016.1
(22) Anmeldetag: 16.05.2006
(51) Int. Cl.: B01D 3/00, B01J 19/00, C07C 17/02

(54) **Verbesserte Prozessführung bei der Direktchlorierung des Ethylens zu 1,2-Dichlorethan**
Improved process for direct chlorination of ethylene to 1,2-dichloroethane
Procédé amélioré pour la fabrication de 1,2-dichloréthane par chloration directe de l'ethylene

(43) Veröffentlichungstag der Anmeldung: 21.11.2007
(73) Patentinhaber: VESTOLIT GmbH & Co. KG, 45772 Marl (DE)
(72) Erfinder: Kahsnitz, John, 45721 Haltern (DE); Polte, Dieter, 46514 Schermbeck (DE)
(74) Vertreter: Godemeyer Blum Lenze Patentanwälte Partnerschaft mbB - werkpatent

(56) Entgegenhaltungen:
- WO-A-00/55107
- US-A- 4 774 372

## Beschreibung

Die vorliegende Erfindung bezieht sich auf ein verbessertes Verfahren zur Herstellung von Dichlorethan und eine Anordnung zur Durchführung des Verfahrens.

Bei der Herstellung von Dichlorethan werden zwei Hauptsyntheserouten unterschieden. Während in dem sogenannten Direktchlorierungsprozess (DC) das Produkt aus Ethylen und Chlor hergestellt wird, entsteht bei dem sogenannten Oxychlorierungsprozess (OC) das gleiche Produkt aus HCl, O₂ und Ethylen. Der Sauerstoff ist im letztgenannten Verfahren nicht nur aus reaktionstechnischen Gründen notwendig, sondern dient auch der Reaktivierung und dem Erhalt der Rieselfähigkeit des eingesetzten Katalysators.

Hauptanwendungsgebiet des so erzeugten Dichlorethans ist die Weiterverarbeitung durch thermische Zersetzung zu Vinylchlorid (VCM) und gasförmigem Chlorwasserstoff (HCl). Das Vinylchlorid dient als Ausgangsstoff für die Polymerisation zum Polyvinylchlorid (PVC). Das erzeugte HCl wird häufig in einer Oxychlorierungsanlage weiterverarbeitet. Der abgestimmte Einsatz aller drei Reaktionsschritte stellt den häufig zitierten "Balanced Process" dar.

Bei dem Direktchlorierungs-Prozess unterscheidet man zwischen einem Niedertemperatur-Verfahren (Low Temperature Chlorination (LTC)) und einem Hochtemperatur-Verfahren (High Temperature Chlorination (HTC)).

Während das LTC-Verfahren bei 20 - 70 °C betrieben wird, läuft das HTC-Verfahren bei Temperaturen von 85 - 200 °C.

Das LTC-Verfahren hat den Vorteil, selektiver zu arbeiten, ist aber dafür energetisch deutlich ungünstiger. So muss beispielsweise für die nachgeschaltete Destillation zusätzlich Wärme zugeführt werden.

Das HTC-Verfahren hingegen ist prinzipiell etwas unselektiver, nutzt dafür aber die Reaktionswärme, um die der Reaktion nachgeschaltete destillative Trennung zu unterstützen.

Durch den Einsatz von sehr selektiven Katalysatoren ist es heutzutage inzwischen möglich, auch den HTC-Prozess bezüglich der Selektivität in den Bereich des LTC-Verfahrens zu bringen (> 99 %).Daher wird heute praktisch ausschließlich der HTC-Prozess technisch realisiert.

Aber auch im HTC-Prozess sind noch weitere Verbesserungen möglich. Da die Reaktionswärme um einen Faktor von ca. 6 mal größer ist als die Verdampfungsentalphie, wird nicht die gesamte entstehende Reaktionswärme für die nachfolgende Destillation benötigt. Auch einfache Destillationseinrichtungen schaffen mit einem Rücklaufverhältnis von ∼ 1 eine Dichlorethan-Reinheit von > 99,9 %. Es bleibt somit mehr als die Hälfte der Reaktionswärme ungenutzt. Diese Wärmemenge kann einer sinnvollen Verwendung innerhalb oder außerhalb des Prozesses zugeführt werden.

Um eine hohe Ausbeute an Dichlorethan sicherzustellen, sollte die Folgereaktion zu höher chlorierten Verbindungen möglichst unterbunden werden. Hierzu wird das Ethylen bevorzugt möglichst intensiv mit dem anderen Edukt Chlor in Verbindung gebracht. Der unvorteilhafte Kontakt zwischen dem eingesetzten Lösemittel Dichlorethan und dem Chlor ist dementsprechend möglichst genau einzustellen und auf ein Minimum zu begrenzen.

In einigen Schriften (z.B. DE 4 133 810 A1, DE 0 080 098, EP-A 0 471 987) wird entgegen diesem Kenntnisstand zunächst das Chlor und das Ethylen dosiert, was sicherlich zur vermehrten Bildung einer unerwünschten Menge von Hochsiedern führt.

Auch die Reaktionsführung wird im Stand der Technik häufig beschrieben. Hier wird es teilweise als notwendig erachtet, die Reaktion im Kreislaufreaktor durchzuführen, um eine zufriedenstellende Ausbeute zu erreichen (DE 0 080 098). Bei Anwendung des hier beschriebenen erfindungsgemäßen Verfahrens scheint diese Notwendigkeit jedoch nicht zu bestehen.

Ein weiteres Thema ist die während der Reaktion erzeugte Abwärme. Hier wird z.B. in der Anmeldung DE-A 199 10 964 die Kombination von "Kreislauffahrweise" und Abwärmenutzung beschrieben.

US 4,774,372 offenbart ein Verfahren zur Herstellung von Dichloroethan aus Ethylen und Chlor in hoher Selektivität.

Aufgabe der vorliegenden Erfindung war eine verbesserte und individuellere Verfahrensführung des Direktchlorierungsprozesses und eine bessere Nutzung der entstehenden Energie.

Diese Aufgabe wird gelöst durch ein Verfahren zur Herstellung von 1,2-Dichlorethan (DCE), umfassend das Einspeisen von Ethylen und Chlor in einen mit Dichlorethan gefüllten Reaktor mit aufgesetzter Destillationskolonne und einem System zur Wärmerückgewinnung, wobei die Umsetzung von Ethylen und Chlor zu Dichlorethan bei einer Temperatur von 80 bis 130 °C und einem absoluten Druck von 0,8 bis 5 bar erfolgt, das Reaktionsgemisch am Sieden gehalten wird und die Reaktionswärme aus dem Reaktionsgemisch mit Hilfe zumindest eines Wärmetauschers partiell entfernt, dadurch gekennzeichnet, dass das Verfahren mit einer Vorrichtung durchgeführt wird, die (a) als Reaktor einen Reaktor R1 mit einer Anordnung eines externen Kreislaufes P1 mit einem Wärmetauscher W1 und (b) als aufgesetzte Destillationskolonne eine Kolonne K1 mit einer Anordnung eines externen Kreislaufes mit einem Wärmetauscher W2 umfasst.

Gegenstand der vorliegenden Anmeldung ist auch eine Vorrichtung zur Durchführung eines Verfahrens, dadurch gekennzeichnet, dass sie
(a) einen Reaktor R1 mit einer Anordnung eines externen Kreislaufes P1 mit einem Wärmetauscher W1 und
(b) eine aufgesetzte Destillationskolonne K1 mit einer Anordnung eines externen Kreislaufes mit einem Wärmetauscher W2 umfasst.

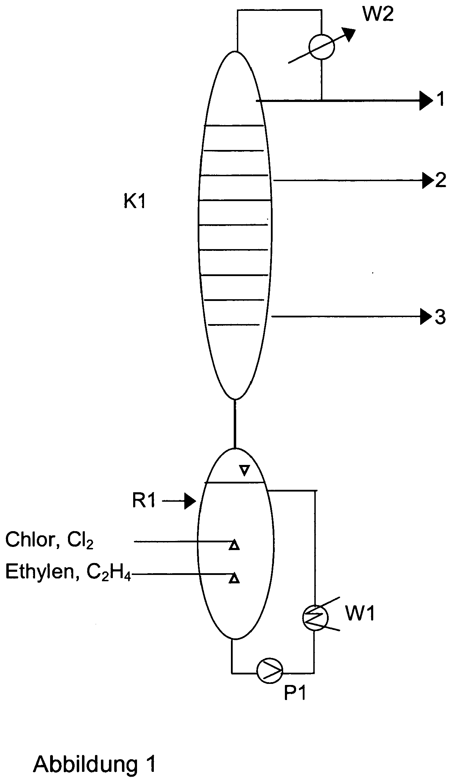

Gegenstand der vorliegenden Anmeldung ist auch ein Verfahren zur Direktchlorierung von Ethylen zu Dichlorethan unter Einsatz eines Reaktors, der eine serielle Ethylen- und Chlor-Zudosierung ermöglicht, mit einer auf den Reaktor aufgesetzten Kolonne und wenigstens einer externen Wärmeabfuhr wie in Abbildung 1 dargestellt. Die Reaktion kann gemäß der Erfindung in einem Reaktor R1 unter der seriellen Zugabe der Edukte Ethylen und Chlor unter Verwendung von Dichlorethan als Lösungsmittel und einem Katalysator stattfinden. Durch die lokal entstehende Reaktionswärme tritt in dem Reaktor eine durch Dichteunterschiede induzierte aufwärtsgerichtete Strömung auf.

Vorteilhaft für eine gute Selektivität bezüglich Dichlorethan ist es, das Ethylen in das Reaktionsgemisch (Dichlorethan und Katalysator) gelöst oder zumindest hochdispers einzubringen. So steht für das anschließend eingebrachte Chlor immer ein gewünschter Reaktionspartner zur Verfügung. Steht dagegen kein freies Ethylen zur Verfügung, kann das Chlor mit dem Dichlorethan im Reaktionsgemisch zu Hochsiedern umgesetzt werden.

Die Reaktionspartner Ethylen und Chlor können durch inerte Gase verdünnt sein. Als Katalysator empfiehlt sich die Verwendung von mit Natriumchlorid modifiziertem Eisen-III-Chlorid und als Inhibitor zur Vermeidung der Nebenproduktbildung wird vorzugsweise Sauerstoff verwendet.

Der Umlauf des flüssigen Mediums im Reaktor kann beispielsweise mittels Pumpe und/oder nach dem Thermosiphon- bzw. Mammutpumpenprinzip bewirkt werden. Die Umlaufgeschwindigkeit des flüssigen Mediums soll in der Mischzone nicht kleiner als 0,1 m/sec sein. Der Umlauf zum Wärmeaustausch und zur Produktverdampfung kann ebenfalls mittels Pumpe und/oder nach dem Thermosiphonprinzip erfolgen, es ist sogar möglich, beide Vorgänge hintereinander geschaltet in einem einzigen Kreislauf flüssigen Mediums ablaufen zu lassen.

Bei typischen Reaktionsbedingungen von Temperaturen von 116 °C und Absolutdrücken von 2 bar lassen sich so Reaktionsselektivitäten von 99,7 % erzielen.

Die hier angegebenen Reaktionsbedingungen dienen als Beispiel und beschreiben eine bevorzugte Ausführungsform der Erfindung, sollen jedoch nicht so angesehen werden, dass sie die Erfindung einschränken.

Die aufgesetzte Kolonne K1 bietet den Vorteil die im Reaktor R1 entstehende Reaktionswärme unmittelbar ohne Wärmeaustausch für die der Reaktion nachfolgende Destillation einsetzen zu können. So können Leichtsieder und flüchtige Komponenten über die Leitung 1, das gereinigte Fertigprodukt Dichlorethan über die Leitung 2 und Hochsieder über die Leitung 3 aus dem Prozess entfernt werden.

Über einen Kreislauf P1 kann die Reaktionswärme aus dem Reaktor R1 teilweise an den Wärmetauscher W1 abgegeben werden. Dieser Wärmetauscher W1 kann die Wärme beispielsweise entweder auf ein anderes Prozessmedium aus derselben oder einer anderen Anlage übertragen, das für seinen Einsatz in diesem oder einem anderen Verfahren vorgewärmt werden muss, oder alternativ kann Wasser zu Niederdruckdampf oder zu Warmwasser verarbeitet und einer sinnvollen energetischen Nutzung zugeführt werden.

Als Umwälzorgan P1 kann auch ein unter Überdruck stehender Ethylen-betriebener Gasstrahler eingesetzt werden. Hier gibt es keine bewegten Teile, die durch Kavitation geschädigt werden können und es findet eine intensive Vermischung des Reaktionsgemisches mit Ethylen statt, welches einen positiven Einfluss auf die Selektivität hat.

Durch die Kombination der auf dem Reaktor R1 aufgesetzten Destillationskolonne K1 und den externen Wärmetauschern W1 und optional W2 zur Wärmerückgewinnung lassen sich viele Betriebszustände des Reaktors einstellen. Bei einer Fahrweise mit einer hohen Dichlorethan-Selektivität der Reaktion und/oder geringen Erwartungen an die Dichlorethan-Reinheit kann viel Wärme über den Wärmetauscher W1 abgeführt und weitergenutzt werden. Läuft die Reaktion nur mit geringer Dichlorethan-Selektivität ab, oder soll Dichlorethan höherer Reinheit über Leitung 2 abgegeben werden, wird bevorzugt mehr Wärme über den Wärmetauscher W2 am Kopf der Destillationskolonne K1 abgeführt und weniger über den Wärmetauscher W1. Durch die Wärmeentnahme im Destillationskopf erhöht sich das Rücklaufverhältnis in der Destillationskolonne und ihre Reinigungswirkung wird besser.

Beispielsweise ist eine sehr hohe Dichlorethan-Reinheit (> 99,9 %) erforderlich, um eine russarme Weiterverarbeitung im Dichlorethan-Cracker zum VCM zu gewährleisten. Ursache für das Erfordernis einer höheren Aufreinigung kann der Einsatz von unreinerem Ethylen oder können Reaktionsprobleme z.B. mit dem im Reaktor R1 vorgelegten Katalysator sein.

### Abbildungen:

Abbildung 1 zeigt eine Anordnung einer Vorrichtung zur Durchführung des Verfahrens der vorliegenden Erfindung mit einem Reaktor R1, in den die Edukte der Reaktion eingespeist werden, der einen externen Kreislauf P1 mit einem Wärmetauscher W1 aufweist, über den Reaktionswärme aus dem Reaktor abgeführt werden kann, und der mit einer Kolonne K1 bestückt ist. Auch die Kolonne K1 kann einen externen Kreislauf mit einem Wärmetauscher W2 umfassen, über den überschüssige Wärme abgeführt werden kann. Über die Ableitungen 1, 2 und 3 der Kolonne können verschiedene Reaktionsprodukte der in dem Reaktor ablaufenden Reaktion abgeführt werden.

### Beispiele:

### Beispiel 1:

In einen Reaktor R1 der Bauweise, wie in Abbildung 1 gezeigt (V = 20 m³, T = 116 °C, p = 2 bar abs.) werden 4.000 Nm³/h Cl₂ und ein entsprechender Mengenstrom Ethylen eingespeist. Als Reaktionsgemisch wurden 18 m³ Dichlorethan-/KatalysatorGemisch eingesetzt. Als Katalysator wurde ein Natriumchlorid-modifiziertes Eisen-III-Chlorid verwendet. Die Reaktionsselektivität liegt bei 99,7 %. Eine auf dem Reaktor aufgesetzte Kolonne K1 besteht aus 25 Böden und umfasst drei Ableitungen. Insgesamt wird eine Reaktionswärme von ca. 11 MW Leistung erzeugt. Wird im Sumpfbereich des Reaktors über den Wärmetauscher W1 eine Wärme von 1 MW Leistung abgeführt, kann eine Dichlorethan-Produktreinheit von 99,99 Gew-% erreicht werden.

### Beispiel 2:

In einem Reaktor R1 der Bauweise, wie in Abbildung 1 gezeigt (V = 20 m³, T = 116 °C, p = 2 bar abs.) werden 4.000 Nm³/h Cl₂ und ein entsprechender Mengenstrom Ethylen eingespeist. Als Reaktionsgemisch wurden 18 m³ Dichlorethan-/KatalysatorGemisch eingesetzt. Als Katalysator wurde ein Natriumchlorid-modifiziertes Eisen-III-Chlorid verwendet. Die Reaktionsselektivität liegt bei 99,7 % Die aufgesetzte Kolonne K1 besteht aus 25 Böden und umfasst drei Ableitungen. Insgesamt wird eine Reaktionswärme von ca. 11 MW Leistung erzeugt. Wird im Sumpfbereich des Reaktors über den Wärmetauscher W1 eine Wärme von 3,5 MW Leistung abgeführt, kann eine Dichlorethan-Produktreinheit von 99,95 Gew-% erreicht werden.

## Patentansprüche

1. Verfahren zur Herstellung von 1,2-Dichlorethan, umfassend das Einspeisen von Ethylen und Chlor in einen mit Dichlorethan gefüllten Reaktor mit aufgesetzter Destillationskolonne und einem System zur Wärmerückgewinnung, wobei die Umsetzung von Ethylen und Chlor zu Dichlorethan bei einer Temperatur von 80 bis 130 °C und einem absoluten Druck von 0,8 bis 5 bar erfolgt, das Reaktionsgemisch am Sieden gehalten wird und die Reaktionswärme aus dem Reaktionsgemisch mit Hilfe zumindest eines Wärmetauschers partiell entfernt wird, **dadurch gekennzeichnet, dass** das Verfahren mit einer Vorrichtung durchgeführt wird, die
(a) als Reaktor einen Reaktor R1 mit einer Anordnung eines externen Kreislaufes P1 mit einem Wärmetauscher W1 und
(b) als aufgesetzte Destillationskolonne eine Kolonne K1 mit einer Anordnung eines externen Kreislaufes mit einem Wärmetauscher W2 umfasst.

2. Verfahren nach Anspruch 1, **dadurch gekennzeichnet, dass** die Destillationskolonne mit der Reaktionswärme aus dem Reaktor betrieben wird.

3. Verfahren nach Anspruch 1 oder 2, **dadurch gekennzeichnet, dass** die Wärmeentnahme aus dem Reaktionsgemisch nach den Erfordernissen der Wärmeeinbringung in die Destillationskolonne ausgerichtet wird.

4. Verfahren nach Anspruch 1, **dadurch gekennzeichnet, dass** die Reaktionswärme einer weiteren Nutzung zugeführt wird.

5. Verfahren nach einem der Ansprüche 1 bis 4, **dadurch gekennzeichnet, dass** die Wärmeentnahme aus dem Reaktionsgemisch mittels eines Kreislaufs über einen externen Wärmetauscher realisiert wird.

6. Verfahren nach Anspruch 5, **dadurch gekennzeichnet, dass** die Reaktionswärme genutzt wird, um andere Stoffströme vorzuwärmen oder ein universell einsetzbares Kreisheizmedium wie Warmwasser oder Dampf zu erzeugen.

7. Vorrichtung zur Durchführung eines Verfahrens nach einem der Ansprüche 1 bis 6, **dadurch gekennzeichnet, dass** sie
(a) einen Reaktor R1 mit einer Anordnung eines externen Kreislaufes P1 mit einem Wärmetauscher W1 und
(b) eine aufgesetzte Destillationskolonne K1 mit einer Anordnung eines externen Kreislaufes mit einem Wärmetauscher W2 umfasst.

8. Vorrichtung gemäss Anspruch 7, **dadurch gekennzeichnet, dass** Einbauten in dem Reaktor vorhanden sind, wobei die Einbauten vorzugsweise statische Mischer sind.

9. Vorrichtung gemäss Anspruch 7 oder 8, **dadurch gekennzeichnet, dass** die Edukte der Reaktion über getrennte Öffnungen in den Reaktor zugegeben werden können.

10. Vorrichtung gemäss Anspruch 8, **dadurch gekennzeichnet, dass** Ethylen als erstes in Strömungsrichtung dem Strom zugegeben wird.

11. Vorrichtung gemäss einem der Ansprüche 7 bis 9, **dadurch gekennzeichnet, dass** die Kolonne mehr als eine Entnahmeöffnung aufweist.

## Claims

1. A process for producing 1,2-dichloroethane, comprising feeding ethylene and chlorine into a reactor filled with dichloroethane and having a distillation column fitted thereon and a system for recovery of heat, wherein the conversion of ethylene and chlorine into dichloroethane is performed at a temperature from 80 to 130 °C and an absolute pressure from 0,8 to 5 bar, the reaction mixture is kept boiling and the heat of reaction from the reaction mixture is partially removed by using at least one heat exchanger, **characterized in that** said process is carried out with a device which comprises,
(a) as a reactor, a reactor R1 having an arrangement of an external circuit P1 with a heat exchanger W1, and,
(b) as a distillation column fitted thereon, a column K1 having an arrangement of an external circuit with a heat exchanger W2.

2. The process according to claim 1, **characterized in that** said distillation column is operated with the heat of reaction from the reactor.

3. The process according to claim 1 or 2, **characterized in that** said removal of heat from the reaction mixture is adjusted to the requirements of the input of heat into said distillation column.

4. The process according to claim 1, **characterized in that** the heat of reaction is put to further use.

5. The process according to any one of claims 1 to 4, **characterized in that** said removal of heat from the reaction mixture is carried out by means of a circuit via an external heat exchanger.

6. The process according to claim 5, **characterized in that** the heat of reaction is used to preheat other material flows or to generate a universally applicable circulatory heating medium like hot water or steam.

7. A device for carrying out a process according to any one of claims 1 to 6, **characterized in that** said device comprises
(a) a reactor R1 having an arrangement of an external circuit P1 with a heat exchanger W1, and
(b) a distillation column K1 fitted thereon, having an arrangement of an external circuit with a heat exchanger W2.

8. The device according to claim 7, **characterized in that** installations are present inside the reactor, wherein said installations are preferably static agitators.

9. The device according to claim 7 or 8, **characterized in that** the starting materials of the reaction can be added to the reactor via separate openings.

10. The device according to claim 8, **characterized in that** ethylene is added first to the flow in the direction of flow.

11. The device according to any one of claims 7 to 9, **characterized in that** said column has more than one withdrawal opening.

## Revendications

1. Procédé servant à fabriquer du 1,2-dichloroéthane, comprenant l'injection d'éthylène et de chlore dans un réacteur rempli de dichloroéthane avec une colonne de distillation érigée et un système servant à la récupération de chaleur, dans lequel la transformation de l'éthylène et du chlore en dichloroéthane a lieu à une température de 80 à 130 °C et à une pression absolue de 0,8 à 5 bar, le mélange réactionnel continu à être réchauffé et la chaleur de réaction issue du mélange réactionnel est supprimée en partie à l'aide au moins d'un échangeur de chaleur, **caractérisé en ce que** le procédé est effectué avec un dispositif, qui comprend
(a) en tant que réacteur un réacteur R1 avec un ensemble d'un circuit externe P1 avec un échangeur de chaleur W1 et
(b) en tant que colonne de distillation érigée une colonne K1 avec un ensemble d'un circuit externe avec un échangeur de chaleur W2.

2. Procédé selon la revendication 1, **caractérisé en ce que** la colonne de distillation fonctionne avec la chaleur de réaction issue du réacteur.

3. Procédé selon la revendication 1 ou 2, **caractérisé en ce que** le prélèvement de chaleur du mélange réactionnel répond aux critères de l'apport de chaleur dans la colonne de distillation.

4. Procédé selon la revendication 1, **caractérisé en ce que** la chaleur de réaction est amenée à une autre utilisation.

5. Procédé selon l'une quelconque des revendications 1 à 4, **caractérisé en ce que** le prélèvement de chaleur du mélange réactionnel est réalisé au moyen d'un circuit par l'intermédiaire d'un échangeur de chaleur externe.

6. Procédé selon la revendication 5, **caractérisé en ce que** la chaleur der réaction est utilisée afin de préchauffer d'autres flux de matières ou de produire un milieu de chauffage à usage universel tel que de l'eau chaude ou de la vapeur.

7. Dispositif servant à mettre en oeuvre un procédé selon l'une quelconque des revendications 1 à 6, **caractérisé en ce qu'**il comprend
(a) un réacteur R1 avec un ensemble d'un circuit externe P1 avec un échangeur de chaleur W1 et
(b) une colonne de distillation K1 érigée avec un ensemble d'un circuit externe avec un échangeur de chaleur W2.

8. Dispositif selon la revendication 7, **caractérisé en ce que** des composants sont présents dans le réacteur, dans lequel les composants sont de préférence des mélangeurs statiques.

9. Dispositif selon la revendication 7 ou 8, **caractérisé en ce que** les produits de départ de la réaction peuvent être ajoutés par l'intermédiaire d'ouvertures séparées dans le réacteur.

10. Dispositif selon la revendication 8, **caractérisé en ce que** l'éthylène est ajouté en premier au flux dans la direction d'écoulement.

11. Dispositif selon l'une quelconque des revendications 7 à 9, **caractérisé en ce que** la colonne présente plus d'une ouverture de prélèvement.
